(19) Europäisches Patentamt
European Patent Office
Office européen des brevets

(11) **EP 2 405 253 A1**

(12) **EUROPEAN PATENT APPLICATION**
published in accordance with Art. 153(4) EPC

(43) Date of publication:
**11.01.2012 Bulletin 2012/02**

(51) Int Cl.:
***G01N 21/21*** *(2006.01)*   ***A61B 5/1455*** *(2006.01)*

(21) Application number: **09841128.3**

(22) Date of filing: **04.03.2009**

(86) International application number:
**PCT/JP2009/054592**

(87) International publication number:
**WO 2010/100766 (10.09.2010 Gazette 2010/36)**

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(71) Applicants:
• **Global Fiberoptics, Ltd.**
**Tokyo 101-0061 (JP)**
• **Shionogi & Co., Ltd.**
**Osaka 541-0045 (JP)**

(72) Inventors:
• **KAJIOKA, Hiroshi**
**Tokyo 102-0082 (JP)**
• **TOTTORI, Yusaku**
**Ageo-Shi**
**Saitama 362-0021 (JP)**

(74) Representative: **Müller-Boré & Partner**
**Patentanwälte**
**Grafinger Straße 2**
**81671 München (DE)**

(54) **OPTICAL ROTATION MEASURING DEVICE AND OPTICAL ROTATION MEASURING METHOD**

(57)    A small-size optical rotation measuring device for detecting an organism, a tissue, blood, or molecules having rotatory power and determining the content thereof with high accuracy and an optical rotation measuring method for detecting an organism, a tissue, blood, or molecules having rotatory power and determining the content thereof with high accuracy. A nonreciprocal optical system is disposed in a loop optical path of a ring optical interferometer, and thereby light beams of circularly polarized modes orthogonal to each other are propagated in opposite directions through a sample to be measured. The wavelength of the light beams from a light source is in a wavelength region where the loss by the nonreciprocal optical element is low. A signal processing technique for phase-modulation optical fiber gyro having the highest resolution among ring interferometers is applied.

[Fig.2]

Printed by Jouve, 75001 PARIS (FR)

## Description

### TECHNICAL FIELD

The present invention relates to an optical rotation measuring device and an optical rotation measuring method for analyzing the optical rotation characteristics of a specimen, and detecting existence of a living body, tissue, blood, a molecule, etc., which have rotary polarization, and content thereof with high precision. More specifically, it relates to an optical rotation measuring device and an optical rotation measuring method for measuring the specific optical rotation of a substance with rotary polarization included in blood, saliva, hair and a specific living tissue of a human body with high precision.

### BACKGROUND ART

There are three main conventional optical methods for measuring blood sugar level. The first method is a method of irradiating an infrared laser beam on a part of a living body such as a finger dispersing the scattered light from a blood vessel, and measuring glucose in the blood, as described in Patent Document 1. This method utilizes the fact that the scattered light decreases in proportion to the glucose concentration. This method has a problem that the light intensity of the scattered light is dependent on temperature, moisture and oil component of the skin etc., and is therefore not popular in actuality.

The second method is a method of making the polarized-light component orthogonal to glucose transmit, and then measuring a birefringence in an open loop, as described in Non-patent Document 1 and Patent Document 2, etc. However, according to this method, error is large when 0.1 g/ dL, which is a healthy person's blood sugar level, is measured using an approximately 10 mm long specimen (glucose.) That is, according to this method, low-inversive or non-invasive measurement of a 1 mm or less long specimen, through which a sufficient quantity of a transmitted light is obtained for an examination of glucose concentration in whole blood, runs short of precision considerably.

The third method is a method of measuring using the birefringence measuring device described in Patent Document 3. This method uses a nonreciprocal optical system deployed in an interferometer ring as with the present invention, and measures a specific optical rotation of a subject fixed inside; wherein a 800 nm band in wave length is used as a light source in the embodiment. This method could not provide sufficient measurement accuracy for noninvasive measurement of a living body, such as a minute amount of blood in a 1 mm or less thick specimen or a 0.1 mm-thick blood vessel although 0.1 g/ dL, which is a healthy person's blood sugar level, can be measured with sufficient precision for 10mm-thick glucose. Moreover, there is a problem that an insertion loss of 6 dB occurs with an optical directional coupler, which separates and couples light source and photo detector, and thus the optical output level of the interferometer is low.

Patent Document 1: JP 2004-313554
Patent Document2: JP 2007-093289
Patent Document 3: JP 2005-274380 (Patent application laid-open 2004-088544)
Non-patent Document 1: Yokota Masayuki at el., "Glucose sensor using a lead glass fiber polarization modulation device", The 31st lightwave sensing technical study meeting LST 31-8, PP. 51 - 56, August, 2003.
Non-patent Document 2: Kajioka and Oho, "Development of optical fiber gyro", The third lightwave sensing technical study meeting, LST 3-9, PP. 55 - 62, June, 1989.

### DISCLOSURE OF THE INVENTION

#### Problem to be solved by the Invention

An objective of the present invention is to provide an optical rotation measuring device and an optical rotation measuring method for detecting existence of a living body, tissue, blood, a molecule etc., and content thereof with high precision, wherein the optical rotation measuring device is considerably improved in sensitivity as compared to the conventional optical rotation measuring device.

#### Means of Solving the Problem

The optical rotation measuring device and the optical rotation measuring method according to the present invention, which are invented so as to solve the problem, feature a method of detecting a phase difference, which is Sagnac phase difference, by deploying a nonreciprocal optical system in a sensing loop of an all polarization-preserving optical fiber gyro on the phase modulation basis, making a right-handed and a left-handed circulary light to propagate through a specimen in the form of polarized waves, and then detecting the phase difference; wherein
a low loss optical circulator is used as the first directional coupler and the wave length of a light source is set to a wavelength band where insertion loss of a Faraday rotation nonreciprocal element of the nonreciprocal optical system is low.

Detailed embodiments are described hereafter. According to a first aspect of the present invention (Hereafter, referred to as the first aspect) invented so as to solve the problem, an optical rotation measuring device is **characterized in that** it includes a nonreciprocal optical system that is deployed in a ring of a ring interferometer and propagates a right-handed circulary light and a left-handed circulary light in the form of mutually orthogonal polarized waves;
a specimen mounting unit deployed in the nonreciprocal optical system, wherein a specimen of whole blood having birefringence or optical rotation, centrifuged blood, a molecule, saliva, a living tissue such as hair, or a cell is mounted on the specimen mounting unit; and
a measuring unit for measuring a phase difference between the propagated, right-handed circulary light and the propagated, left-handed circulary light traveling through the ring; wherein wave length of a light source is 1,300 nm or greater and 1700 nm or less.

According to a second aspect of the present invention (Hereafter, referred to as the second aspect) based on optical rotation measuring device of the first aspect, the optical rotation measuring device according to the second aspect is **characterized in that** the ring interferometer is a phase-modulation based interferometer comprising an all polarization-preserving fiber and associated parts; and the right-handed circulary light and the left-handed circulary light are propagated in the same intrinsic polarization mode of the ring of the polarization-preserving fiber except for the nonreciprocal optical system.

According to a third aspect of the present invention (Hereafter, referred to as the third aspect) based on optical rotation measuring device of either the first or the second aspect, the optical rotation measuring device according to the third aspect is **characterized in that** an optical circulator is used for a coupler of the ring interferometer.

According to a fourth aspect of the present invention (Hereafter, referred to as the fourth aspect) based on optical rotation measuring device of any one of the first to the third aspect, the optical rotation measuring device according to the fourth aspect is **characterized in that** a collimated space propagating beam is optimized, so as for an opposing coupling loss of the nonreciprocal optical system plus an absorption and a scattering loss of a living body to be approximately 40 dB or less.

According to a fifth aspect of the present invention (Hereafter, referred to as the fifth aspect) based on optical rotation measuring device of any one of the first to the fourth aspect, the optical rotation measuring device according to the fifth aspect is **characterized in that** the distance of a space propagation portion in which a specimen is shut in is variable, where the space propagation portion is for the opposing collimators in the nonreciprocal optical system, which sandwich a part of a living body as the specimen.

According to a sixth aspect of the present invention (Hereafter, referred to as the sixth aspect) based on optical rotation measuring device of any one of the first to the fifth aspect, the optical rotation measuring device according to the sixth aspect is **characterized in that** it further includes
an analyzing unit for measuring a wavelength property at a measured phase angle, conducting numerical analysis of the wavelength property, and qualitatively and/or quantitatively estimating existence of the specimen and content of the specimen; wherein the light entering the ring interferometer is variable in wavelength.

According to a seventh aspect of the present invention (Hereafter, referred to as the seventh aspect) based on optical rotation measuring device of any one of the first to the sixth aspect, the optical rotation measuring device according to the seventh aspect is **characterized in that** it further includes a living body holding unit for pressuring and shutting in a subject of a living body, wherein the living body holding unit is deployed in the opposing collimators.

According to an eighth aspect of the present invention (Hereafter, referred to as the eighth aspect) invented so as to solve the problem, an optical rotation measuring method is **characterized in that** it includes using: a nonreciprocal optical system that is deployed in a ring of a ring interferometer and propagates a right-handed circulary light and a left-handed circulary light in the form of mutually orthogonal polarized waves; a specimen mounting unit deployed in the nonreciprocal optical system, wherein the specimen of whole blood having birefringence or optical rotation, centrifuged blood, a molecule, saliva, a living tissue such as hair, or a cell is mounted on the specimen mounting unit, and a measuring unit for measuring a phase difference between the propagated, right-handed circulary light and the propagated, left-handed circulary light traveling through the ring, wherein wavelength of a light source is 1,300 nm or greater and 1700 nm or less; and detecting existence of a specimen and content of the specimen according to the result from the measuring unit.

According to a ninth aspect of the present invention (Hereafter, referred to as the ninth aspect) based on optical rotation measuring method of the eighth aspect, the optical rotation measuring method according to the ninth aspect is **characterized in that** the ring interferometer is a phase-modulation based interferometer comprising an all polarization-preserving fiber and associated parts; and the right-handed circulary light and the left-handed circulary light are propagated in the same intrinsic polarization mode of the ring of the polarization-preserving fiber except for the nonreciprocal optical system.

According to a tenth aspect of the present invention (Hereafter, referred to as the tenth aspect) based on optical rotation measuring method of either the eighth or the ninth aspect, the optical rotation measuring method according to the tenth aspect is **characterized in that** an optical circulator is used for a coupler of the ring interferometer.

According to an eleventh aspect of the present invention (Hereafter, referred to as the eleventh aspect) based on optical rotation measuring method of any one of the eighth to the tenth aspect, the optical rotation measuring method according to the eleventh aspect is **characterized in that** a collimated space propagating beam is optimized, so as for an opposing coupling loss of the nonreciprocal optical system plus an absorption and a scattering loss of a living body to be approximately 40 dB or less.

According to a twelfth aspect of the present invention (Hereafter, referred to as the twelfth aspect) based on optical rotation measuring method of any one of the eighth to the eleventh aspect, the optical rotation measuring method according to the twelfth aspect is **characterized in that** the distance of a space propagation portion in which a specimen is shut in is variable, where the space propagation portion is for the opposing collimators in the nonreciprocal optical system, which sandwich a part of a living body as the specimen.

According to a thirteenth aspect of the present invention (Hereafter, referred to as the thirteenth aspect) based on optical rotation measuring method of any one of the eighth to the twelfth aspect, the optical rotation measuring method according to the thirteenth aspect is **characterized in that** it further includes measuring a wavelength property at a measured phase angle; conducting numerical analysis of the wavelength property; and qualitatively and/or quantitatively estimating existence of the specimen and content of the specimen; wherein the light entering the ring interferometer is variable in wavelength.

According to a fourteenth aspect of the present invention (Hereafter, referred to as the fourteenth aspect) based on optical rotation measuring method of any one of the eighth to the thirteenth aspect, the optical rotation measuring method according to the fourteenth aspect is **characterized in that**

a living body holding unit for pressuring and shutting in a subject of a living body is deployed in the opposing collimators.

Results of the invention

The first result of the present invention is that measurement of the optical rotation with very high precision is possible because it utilizes the optical interference principle. The second result is that since the wavelength of a light source for an optical interferometer is set to be in a wavelength band that causes an insertion loss of a nonreciprocal optical system to which a specimen is inserted and loss of a directional coupler, which separates and couples the light source and a photo detector, to be lower. The light receiving power is improved to be approximately 1,000 times, allowing measurement of the specific optical rotation of a living body, such as an extremely minute specimen, finger, ear, and webbing between the thumb and the index finger, with extremely high precision as compared to the conventional method. These advanced constituent elements allow provision of a non-invasive, or low-invasive optical rotation measuring device for a living body with substantially higher precise than before.

BRIEF DESCRIPTION OF DRAWINGS

Fig. 1 is an illustration of an entire configuration specifying an optical rotation measuring device according to an embodiment of the present invention;
Fig. 2 is a detailed block diagram of the optical rotation measuring device according to the embodiment of the present invention;
Fig. 3 is a block diagram of a nonreciprocal optical system of the optical rotation measuring device according to the embodiment of the present invention; and
Fig. 4 is a block diagram illustrating an example of an experiment for measurement of the optical rotation of a living body according to the embodiment of the present invention.

Description of Reference Numerals

1: Light Source (ASE)
2: Optical Interferometer
3, 11-1, 11-2: Nonreciprocal optical system
4: Optical fiber gyro phase detector
5: Optical circulator
6, 13-1, 13-2: Polarizer
7: Optical coupler
8: Polarization-preserving optical fiber
8-1: Polarization-preserving dummy optical fiber
9: Phase Modulator
10-1, 10-2: Lens
12: Specimen to be measured
13: Modulating signal
14-1, 14-2: 45-degree Faraday rotator
15-1, 15-2: 1/4 wave plate
16: Photo detector

DESCRIPTION OF EMBODIMENTS

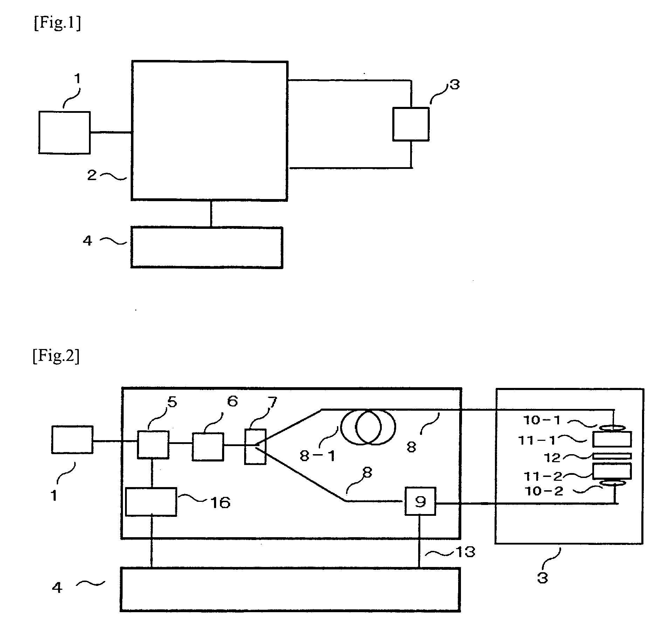

An embodiment is described with reference to Figs. 1 to 3. Description of an optical rotation measuring device according to the present invention may also serve as that of an optical rotation measuring method, or vice versa, in order to avoid redundancy of description thereof while keeping understandability and avoiding misunderstanding. Fig. 1 is a block diagram of a basic constitution according to the present invention. The entire configuration includes a light source 1, an optical interferometer 2, a nonreciprocal optical system 3, and a signal detector 4 of an optical fiber gyro. Fig. 2 describes these elements in detail. A so-called ASE light source of the C band is used as the light source 1, however, an SLD may also be used when required precision is not strict. The light emitted from the light source 1 is branched into a right-handed circulary light and a left-handed circulary light by a coupler 7 via an optical circulator 5 and a polarizer 6. The optical circulator 5 may be constituted by a conventional 2x2 directional coupler, when required precision is not strict. A polarization-preserving fiber 8 is used as an optical path of a ring interferometer. Although an oval-core fiber is used here, a fiber having a core to which an anisotropic stress is applied may also be used.

The branched, clockwise propagating light propagates through the loop of the polarization-preserving fiber 8, passes through a lens 10-1, a nonreciprocal optical system 11-1, and a specimen 12 to be measured, passes through a nonreciprocal optical system 11-2 and a lens 10-2, passes through a phase modulator 9 of the interferometer 2, and returns to the coupler 7. On the other hand, the counter-clockwise propagating light passes through the phase modulator 7 first, propagates through the above-described optical path reversely, and returns to the coupler 7. These clock-wise and counter clock-wise propagating lights interfere with each other at the coupler 7, the interference intensity is changed to an electric signal by a photo detector 16 via the polarizer 6 and the optical circulator 5, and the signal detector 4 of the optical fiber gyro outputs, as a voltage, a phase difference between the clock-wise and counter clock-wise propagating lights. The optical fiber gyro used here is based on the interferometry described in Non-patent Document 2. The loop length is 1000 m. The phase modulator 9 is PZT-based and modulated by a sinusoidal modulating signal 13 of 20-kHz in resonance frequency from the signal detector 4. The optical fiber gyro given in Non-patent Document 2 is a system in which a modulator is modulated by a sinusoidal wave, and a photo detector detects the fundamental frequency component, a second frequency component, and a fourth frequency component. The phase difference is controlled to be a fixed value according to arc tangent (tan-1) of the amplitude ratio of the fundamental frequency component and the second frequency component, and the modulation factor is controlled to be a fixed value according to the ratio of the second frequency component and the fourth frequency component. The RS232C standard is used for electric output of a sensor prototype. However, a commercially available converter or USB may be used for the same. Light-receiving sensitivity is generally also dependent on a modulation factor. The longer the light propagation time propagating the loop, namely, the longer the loop the greater the modulation factor becomes. In this respect, there is a merit of using the C band represented by an optically propagated wave length of 1550 nm.

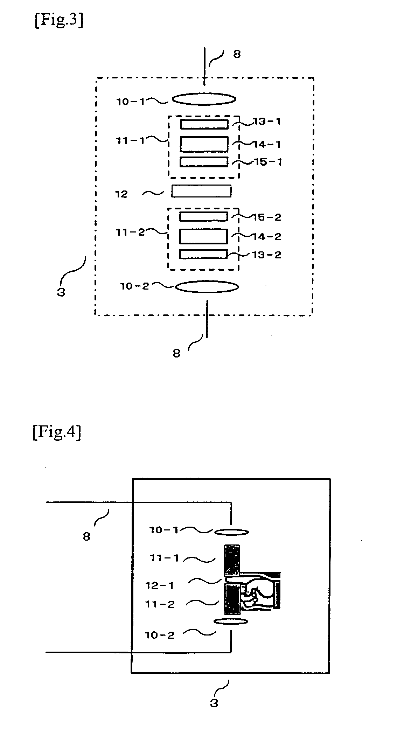

Fig. 3 is a detailed block diagram of the nonreciprocal optical system 3 of Fig. 1. This nonreciprocal optical system is constituted by facing lens 10-1 and 10-2, polarizers 13-1 and 13 -2, 45-degree Faraday rotary elements 14-1 and 14 -2, and 1/4 wave plates 15-1 and 15-2. The Faraday rotary elements are made of iron garnet, and magnets are arranged therearound. The relative angle between the 45-degree Faraday rotary element 14-1 and the 1/4 wave plate 15-1, and relative angle between the 45-degree Faraday rotary element 14-2 and the 1/4 wave plate 15-2 are respectively adjusted so that the right-handed circularly light and the left-handed circularly light passing through specimen 12 can propagate in the forms of a right-handed circularly polarized light and a left-handed circularly polarized light. Such an adjustment allows generation of a phase difference approximately twice the specific angle of an optical rotation generated in a specimen so as to allow measurement thereof by the phase difference detection system of the optical fiber gyro.

In an experiment, a glucose solution injected into a cell is used as the specimen of Fig. 2. In this case, jigs respectively dedicated to a 10 mm x 10 mm cell, a 3 mm x 3 mm cell, and a 1 mm x 1 mm cell are deployed on a stage on which cells are mounted. Reproducibility of measurements is not observed when cells are simply placed manually. However, use of jigs for cell fixation provides reproducibility of the observed values even if cells are detached and attached.

A relationship between the specific optical rotation of glucose and the light-receiving power required therefor is studied here. The blood sugar level of a healthy person's blood is approximately 0.1g/100cc, and the optical rotation angle is approximately 0.005 degrees for sample length L of 10 mm. Therefore, in order to measure glucose contained in an approximately 0.1 mm-thick blood vessel of a human body, it is necessary to measure an extremely minute change in polarization angles: 0.00005 degrees, which is 1/100 of the thickness. As described above, this is equivalent to 0.0001-degree phase change for the ring interferometer.

A S/N ratio of a receive section required for the optical fiber gyro on the phase modulation basis to measure a phase change θ of 0.0001 is studied hereafter.

When the modulation factor is set to be the maximum value, the S/N ratio is approximately expressed by the following equation, as is described in Non-patent Document 2.

$$S/N = \mathrm{Sin}\,(\theta) * \sqrt{(Pr*\eta)}/\sqrt{(2*e*B)} \quad ..... \quad (1)$$

where Pr denotes a light-receiving light power; e denotes charge of an electron ($1.6\times10^{-19}$); and B denotes a receiving bandwidth (which is inversely proportional to the integral time).

When θ= 0.0001 degrees, Pr=1μW, and B= 0.1 Hz (10 seconds) are substituted for that equation, S/N is calculated to be approximately 10. That is, this means that light-receiving power Pr of approximately 1 μW is required for measurement of a phase difference of 0.0001 degrees at a sufficient S/N ratio.

Fig. 4 is illustrative of an example application of the optical rotation measuring device according to the present invention to biopsy. The subject to be measured in this case is the index finger, or webbing 12-1 of the skin between the thumb and the index finger. The experiment is conducted on the webbing. Thickness of the webbing varies person to person, but is generally approximately 3 mm. When collimated lights of the 800 nm band and the 1550 nm band in wave length are made to pass through the webbing, a loss of approximately 10 dB is observed. This is considered to be a total of the absorption loss and scattering loss of the skin. A prime factor accounting for the total loss is the scattering loss by the scattering source of the skin in the 800 nm band, and the other prime factor is the absorption loss by moisture contained in the skin in the 1550 nm band while the scattering loss is low in the 1550 nm band.

An additional important fact found through the experiment is that even approximately 3 mm thin skin scatters the passing collimated light considerably and clear collimated light cannot be obtained. Therefore, when the nonreciprocal optical system is constituted like the present invention by an opposing system of a polarization-preserving fiber, which is a type of single-mode fiber having a small core diameter, the coupling loss is very large. In the measurement system of Fig. 4, a transparent glass plate is adhered to the wave plates 15-1 and 15-2 so that the optical path passing through the webbing is not refracted. Application of an index matching agent to the webbing of a hand is also effective in reduction of the coupling loss. The webbing 12-1 is inserted between the wave plates 15-1 and 15-2 after another alignment apparatus couples opposing collimators beforehand.

In non-invasive measuring of the blood sugar level at an actual medical site, the result of the present invention may be enhanced using a handy tool, which is capable of making an incorporated section of the lens 10-1 and the nonreciprocal optical system 11-1 and incorporated section of the lens 10-2 and the nonreciprocal optical system 11-2 sandwich the specimen 12 or a part of a human body, such as a finger and webbing, by utilizing a spring, etc. while keeping the coupling between the input fiber and the output fiber in the nonreciprocal optical system 3 of Fig. 3. As such an example, opposing collimator devices having variable optical path length claimed in Claim 5 may be fabricated using optical axis adjustment technology.

The loss level of the conventional optical rotation measuring device of the 800 nm band in wave length as described in Patent Document 3 is as follows:

> Light source output: approximately 2 mW
> Optical interferometer loss: approximately 10dB (coupler: 6 dB, polarizer: 3 dB, other: 1 dB) Nonreciprocal optical system loss: 13 dB (the total loss of 10 dB of two Faraday rotary elements is a primary factor)

Therefore, when the skin insertion loss of 10 dB is added thereto, the sum will be 33 dB in total. Furthermore, when the coupling loss of the nonreciprocal optical opposing collimators (> 30 dB) is further added thereto, the total will be 63 dB, which is equivalent to an optical reception of approximately 1 nW. This value is very far from 1 $\mu$W that is required for measurement of a 0.1 mm thick blood vessel.

On the other hand, in the case where the present invention uses as the wave length of the light source the C band represented by the 1550 nm band, which is currently utilized in optical communications, the loss level of an optical rotation measuring device, which is constituted by a light interference system corresponding to the C band, is as follows:

> Light-source output: approximately 100mW (ASE)
> Optical interferometer loss: approximately 5dB (optical circulator: 1 dB, polarizer: 3 dB, and other: 1 dB)
> Nonreciprocal optical system loss: 2 dB (which is small enough to ignore the loss of two Faraday rotary elements)

Therefore, if a skin insertion loss of 10 dB is added thereto, the total will be 17 dB. Moreover, when the coupling loss of the nonreciprocal optical, opposing collimators (> 30 dB) is added thereto, the total will be 47 dB, which is equivalent to an approximately 2 $\mu$W light received. As a result, a required light-receiving level is obtained for measurement of the specific optical rotation of a 0.1 mm-thick blood vessel.

As described above, the light receiving power is improved to be approximately 1,000 times by changing the wave length of the optical interferometer from the 800 nm band to the optical communication wavelength band. A significant change in the phase difference detected by the phase detector 4, which functions as the signal detector 4, is observed between the case where the webbing between the thumb and the index finger of a hand is inserted in the experiment system of Fig. 4 and case where it is not inserted. When the measured region is changed, the phase difference also changes accordingly. This result may be due to dependency on the quantity of the substance having optical rotation in the part through which a light passes. As a result of this inventors' detailed analysis, it is found that a non-invasive optical rotation analysis system according to the present invention may be able to estimate the blood sugar level by measuring a portion providing the greatest phase difference, and creating a measurement model for comparison among measurements of the blood sugar level according to the conventional blood collecting system.

The optical rotation measuring device according to the present invention using the 1550 nm band in optical wave length allows improvement in reception sensitivity by approximately 1,000 times that of the optical rotation measuring device using the 800 nm band in optical wavelength. Therefore, in vitro (blood collecting) based measurement does not require a large quantity of a subject, and dramatically low invasive measurement may be attained.

The specific optical rotation of a glucose solution, blood, plasma, saliva, hair, etc. may be measurable by the optical rotation measuring device according to the present invention. Moreover, since the optical rotation measuring device according to the present invention has a very large light-receiving power, an improved S/N ratio will be provided, thereby shortening the measuring time. Therefore, a merit of easily detecting the phase difference while changing the measured region is provided.

As an embodiment of an optical rotation measuring device according to the present invention, there is a method of constituting optical fibers on either on nonreciprocal optical ends of a nonreciprocal optical system by a polarization-preserving fiber of a photonic crystal type. Use of a photonic crystal fiber may improve the coupling loss of the opposing collimators by approximately 3 dB because use of an expanded core 15 $\mu$m in diameter from the conventional 10 $\mu$m is possible according to the fiber principle.

In addition, according to the embodiment of the optical rotation measuring device of the present invention, two different beams in diameter for a nonreciprocal optical system, the usual 300 $\mu$m and 1 mm for a prototype of this invention, are studied. It is learned that the larger the beam diameter the stricter the relative angle precision of the opposing collimators. However, the larger the diameter, such as 1 mm, the smaller the coupling loss by conducting axis adjustment correctly.

The light receiving sensitivity is substantially improved by shifting the wavelength of the optical rotation measuring device, according to the present invention, to a long wavelength region, and primary factors for such an improvement are summarized below. Firstly, the insertion loss of an iron garnet is very small at 1300 nm or greater due to its own physical property. This garnet type is generally used for the optical isolator and the optical circulator for optical communications. The 1550 nm band, which is advantageous in cost because optical components are widely distributed, is utilized in the embodiment of the present invention. As a result, the insertion loss of the nonreciprocal optical system, which uses two garnets facing each other, decreases, and a low-loss optical circulator using garnets can be replaced for the first coupler of the optical fiber gyro. Secondly, a high-output ASE light source may be used. Thirdly, since the core of the polarization-preserving fiber for the 1550nm band is larger than that of polarization-preserving fiber for the 800 nm band, the coupling loss of the nonreciprocal optical, opposing collimators is small.
Fourthly, even when a longer loop of an optical fiber is used for increasing the modulation factor, the loss can be kept low etc.

The optical rotation measuring device according to the present invention is applied the phase detection principle for the optical fiber gyro on the phase modulation basis, so as to measure a very minute phase difference, such as 0.0001 degrees equivalent to the optical rotation of a 0.1 mm thick blood vessel. The reason why the ring interferometer represented by the optical fiber gyro can measure such a minute phase difference is because lights transmitted bidirectionally have reciprocity in regions other than the nonreciprocal optical system. That is, influence of temperature change and noises such as a vibration is canceled. It is well-known that the optical fiber gyro with a fiber coil 1,000 m in length and 3 cm in diameter for a 1550 nm wave length is generally capable of measuring an angular velocity of 0.1 degrees/second with sufficient precision. In this case, the scale factor (coefficient that will give a phase difference if it is multiplied by angular velocity) will be approximately 1 second. When this is converted to phase difference, it will be equivalent to $2.7 \times 10^{-5}$. Therefore, it is apparent that a phase difference of 0.0001 degrees, which is a target of the present invention, can be surely measured.

The optical rotation measuring device according to the present invention can not separate a substance with multiple optical rotation modes. However, in the case where a substance with an absorption loss at a specific wavelength is contained in a specimen, influence of the substance with a great loss can be separated by changing the optical wavelength and scanning it including an absorption region of the changed wavelength. An equivalent result may be provided even if a wide band light source and a wavelength tunable filter are used instead of the wavelength variable light source. For example, it is known that glucose has an absorption peak in the 1,600 nm band in wave length. According to the embodiment of the present invention claimed in Claim 6, contribution of glucose may be found by scanning wavelengths including 1,600 nm, measuring wavelength property of the phase difference measured by the optical rotation measuring device according to the present invention, and performing numerical calculation.

The optical rotation measuring device and the optical rotation measuring method according to the present invention bring about great improvement in measurement sensitivity using 1300 nm to 1700 nm band in optical wavelength. Furthermore, since the Faraday rotary element, the polarization-preserving fiber, and associated parts used for the optical rotation measuring device of the present invention are popular for optical communications, a cost merit may also be enjoyed.

The optical rotation measuring device and the optical rotation measuring method according to the present invention may provide particularly great results if they are used at a medical site etc. as a living body optical rotation measuring device and a living body optical rotation measuring method.

While the embodiment of the present invention is described with reference to the drawings, the present invention is not narrowly limited to them, and has many possible variations.

Industrial applicability

The optical rotation measuring device and the optical rotation measuring method according to the present invention are capable of investigating a subject having birefringence and optical rotatory power with high precision, and particularly detecting existence of a living body, tissue, blood, a molecule, etc. and content thereof with high precision, and they may be used in medical fields etc. They have the following merits: Firstly, non-invasive measurement of the blood sugar level especially allows a pain-free blood collecting. Secondly, in addition to sanitariness due to no blood collecting, infection via a blood collecting instrument etc. can be prevented. Thirdly, it is economical since no enzyme are used. Fourthly, no waste, such as hypodermic needles and enzymes, is generated.

Note that the third merit is also brought about in invasive measurement by the optical rotation measuring device and optical rotation measuring method according to the present invention.

**Claims**

1. An optical rotation measuring device, comprising: a nonreciprocal optical system that is deployed in a ring of a ring interferometer and propagates a right-handed circulary light and a left-handed circulary light in the form of mutually orthogonal polarized waves;
   a specimen mounting unit deployed in the nonreciprocal optical system, wherein a specimen of whole blood having birefringence or optical rotation, centrifuged blood, a molecule, saliva, a living tissue such as hair, or a cell is mounted on the specimen mounting unit; and
   a measuring unit for measuring a phase difference between the propagated, right-handed circulary light and the propagated, left-handed circulary light traveling through the ring; wherein wave length of a light source is 1,300 nm or greater and 1700 nm or less.

2. The optical rotation measuring device according to Claim 1, wherein the ring interferometer is a phase-modulation based interferometer comprising an all polarization-preserving fiber and associated parts; and
   the right-handed circulary light and the left-handed circulary light are propagated in the same intrinsic polarization mode of the ring of the polarization-preserving fiber except for the nonreciprocal optical system.

3. The optical rotation measuring device according to either Claim 1 or 2, wherein an optical circulator is used for a coupler of the ring interferometer.

4. The optical rotation measuring device according to any one of Claims 1 to 3, wherein a collimated space propagating beam is optimized, so as for an opposing coupling loss of the nonreciprocal optical system plus an absorption and a scattering loss of a living body to be approximately 40 dB or less.

5. The optical rotation measuring device according to any one of Claims 1 to 4, wherein the distance of a space propagation portion in which a specimen is shut in is variable, where the space propagation portion is for the opposing collimators in the nonreciprocal optical system, which sandwich a part of a living body as the specimen.

6. The optical rotation measuring device according to any one of Claims 1 to 5, further comprising:

   an analyzing unit for measuring a wavelength property at a measured phase angle, conducting numerical analysis of the wavelength property, and qualitatively and/or quantitatively estimating existence of the specimen and content of the specimen; wherein the light entering the ring interferometer is variable in wave length.

7. The optical rotation measuring device according to any one of Claims 1 to 6, further comprising a living body holding unit for pressuring and shutting in a subject of a living body, wherein the living body holding unit is deployed in the opposing collimators.

8. An optical rotation measuring method, comprising using: a nonreciprocal optical system that is deployed in a ring of a ring interferometer and propagates a right-handed circulary light and a left-handed circulary light in the form of mutually orthogonal polarized waves; a specimen mounting unit deployed in the nonreciprocal optical system, wherein the specimen of whole blood having double birefringence or optical rotation, centrifuged blood, a molecule, saliva, a living tissue such as hair, or a cell is mounted on the specimen mounting unit, and a measuring unit for measuring a phase difference between the propagated, right-handed circulary light and the propagated, left-handed circulary light traveling through the ring; wherein wave length of a light source is 1,300 nm or greater and 1700 nm or less; and detecting existence of a specimen and content of the specimen according to the result from the measuring unit.

9. The optical rotation measuring method according to Claim 8, wherein the ring interferometer is a phase-modulation based interferometer comprising an all polarization-preserving fiber and associated parts; and the right-handed circulary light and the left-handed circulary light are propagated in the same intrinsic polarization mode of the ring of the polarization-preserving fiber except for the nonreciprocal optical system.

**EP 2 405 253 A1**

**10.** The optical rotation measuring device according to either Claim 8 or 9, wherein an optical circulator is used for a coupler of the ring interferometer.

**11.** The optical rotation measuring method according to any one of Claims 8 to 10, wherein a collimated space propagating beam is optimized, so as for an opposing coupling loss of the nonreciprocal optical system plus an absorption and a scattering loss of a living body to be approximately 40 dB or less.

**12.** The optical rotation measuring method according to any one of Claims 8 to 11, wherein the distance of a space propagation portion in which a specimen is shut in is variable, where the space propagation portion is for the opposing collimators in the nonreciprocal optical system, which sandwich a part of a living body as the specimen.

**13.** The optical rotation measuring method according to any one of Claims 8 to 12, further comprising measuring a wavelength property at a measured phase angle;
conducting numerical analysis of the wavelength property; and qualitatively and/or quantitatively estimating existence of the specimen and content of the specimen; wherein the light entering the ring interferometer is variable in wavelength.

**14.** The optical rotation measuring method according to any one of Claims 8 to 13, wherein a living body holding unit for pressuring and shutting in a subject of a living body is deployed in the opposing collimators.

[Fig.1]

[Fig.2]

[Fig.3]

[Fig.4]

**INTERNATIONAL SEARCH REPORT**

| International application No. |
| --- |
| PCT/JP2009/054592 |

A. CLASSIFICATION OF SUBJECT MATTER
*G01N21/21(2006.01)i, A61B5/1455(2006.01)i*

According to International Patent Classification (IPC) or to both national classification and IPC

B. FIELDS SEARCHED

Minimum documentation searched (classification system followed by classification symbols)
G01N21/21-21/23

Documentation searched other than minimum documentation to the extent that such documents are included in the fields searched
Jitsuyo Shinan Koho          1922-1996   Jitsuyo Shinan Toroku Koho   1996-2009
Kokai Jitsuyo Shinan Koho    1971-2009   Toroku Jitsuyo Shinan Koho   1994-2009

Electronic data base consulted during the international search (name of data base and, where practicable, search terms used)
WPI, JSTPlus(JDreamII), JMEDPlus(JDreamII), JST7580(JDreamII),
UTILITY MODEL FILE(PATOLIS), PATENT FILE(PATOLIS)

C. DOCUMENTS CONSIDERED TO BE RELEVANT

| Category* | Citation of document, with indication, where appropriate, of the relevant passages | Relevant to claim No. |
| --- | --- | --- |
| Y | JP 2005-274380 A  (Optoquest Co., Ltd.), 06 October, 2005 (06.10.05), Full text (Family: none) | 1-3,5-7 |
| Y | JP 2004-113434 A  (Masato NAKAMURA), 15 April, 2004 (15.04.04), Full text (Family: none) | 1-3,5-7 |
| Y | JP 2007-286062 A  (Honeywell International Inc.), 01 November, 2007 (01.11.07), Full text & US 2007/0242276 A1   & EP 1847804 A1 | 3,5-7 |

☒ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 02 April, 2009 (02.04.09) | 21 April, 2009 (21.04.09) |

| Name and mailing address of the ISA/ Japanese Patent Office | Authorized officer |
| --- | --- |
| Facsimile No. | Telephone No. |

Form PCT/ISA/210 (second sheet) (April 2007)

**INTERNATIONAL SEARCH REPORT**

| | International application No. |
| --- | --- |
| | PCT/JP2009/054592 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2007-523356 A  (Honeywell International Inc.), 16 August, 2007 (16.08.07), Full text & JP 2005-524078 A       & US 2004/0165190 A1 & US 2003/0202187 A1     & EP 1718929 A & EP 1499856 A           & WO 2005/083357 A & WO 2003/093764 A1      & CA 2484201 A & BR 309687 A | 3,5-7 |
| Y | JP 2004-77466 A  (Citizen Watch Co., Ltd.), 11 March, 2004 (11.03.04), Full text & US 2004/0036854 A1 | 6,7 |
| Y | JP 2008-113891 A  (Sanyo Electric Co., Ltd.), 22 May, 2008 (22.05.08), Fig. 5 (Family: none) | 5-7 |

Form PCT/ISA/210 (continuation of second sheet) (April 2007)

**INTERNATIONAL SEARCH REPORT**

| International application No. |
| --- |
| PCT/JP2009/054592 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 8-14
   because they relate to subject matter not required to be searched by this Authority, namely:
   The inventions of the claims relate to a diagnostic method practiced on the human body.
   It is obvious that "the subject" stated in claim 8 and "the organism" stated in claims 11, 14 referring to claim 8  (Continued to the extra sheet.)

2. ☒ Claims Nos.: 4, 8-14
   because they relate to parts of the international application that do not comply with the prescribed requirements to such an extent that no meaningful international search can be carried out, specifically:
   The matter that "the whole blood, a centrifugally separated blood, molecules, saliva, an organism tissue such as a hair, or cells" used as a subject is a measurement object "the presence" of which is detected or "the content" of which is determined  (Continued to the extra sheet.)

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

| **INTERNATIONAL SEARCH REPORT** | International application No. |
|---|---|
| | PCT/JP2009/054592 |

Continuation of Box No.II-1 of continuation of first sheet(2)

include a part of a human body since "a part of a human body such as a finger or a rugae" as a measurement object is exemplified in "Best Mode for Carrying out the Invention" of the description on page 5.

The invention of claim 8 includes a step of detecting a subject to be examined which is placed on a subject stage where a subject including a part of a human body is placed or determining the content by using a light source. It should be assumed that the step includes a substep of placing a subject including a part of a human body on a subject stage, applying light to the subject, and performing measurement even though the description fails to explicitly describe the substep. Therefore, the step includes a process on a human body, namely, placement of a human body on a stage, application of light to the human body, and measurement of the applied light exiting from the human body.

In view of the description, the invention of the present application includes at least one working example in which the optical rotation of a part of a human body is measured and the blood sugar level is estimated on the basis of the optical rotation. The blood sugar level is a general index used for check on health condition or disease diagnosis as obvious from the passage "the blood sugar level of a healthy subject" on page 1 of the description.

Claim 8 neither define the invention as a device operating method nor exclude at least the step including the process conducted on a human body by a doctor.


Continuation of Box No.II-2 of continuation of first sheet(2)

is not supported by the description.

The matter is not consistent with the disclosure by the description mainly relating to estimation of the blood sugar level on the basis of the optical rotation of when a part of a human body is used as a subject and containing mere exemplification of measurement of the optical rotation of a glucose solution, blood, plasma, saliva, or a hair. Claims 11, 14 dependent on claims 8 state that "an organism" is a measurement object. However, the relation between "the organism" and "the subject" is not clear, and the invention of claim 8 in which "the subject" is a measurement object is not consistent with the inventions of the other claims.

Therefore, the inventions of claim 8 and the claims dependent on claim 8 are not fully supported by the description and are not defined with a minimum clarity required for the international search.

Claims 4, 11 merely state expected performance wishfully and functionally. It is unclear what constructions as the inventions of a device/method for realizing the performance/function are. In addition, the performance/function is for reducing to a value below a predetermined one the amount of loss by the measurement object such as "an organism" the amount of loss by which is unknown, and the scope of the extent required for the function/performance of the device/method itself of the invention is also unclear.

Consequently, the inventions of claims 4, 11 and the claims dependent on the claims 4, 11 are not defined with a minimum clarity required for the international search.

Form PCT/ISA/210 (extra sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004313554 A
- JP 2007093289 A
- JP 2005274380 A
- JP 2004088544 A

**Non-patent literature cited in the description**

- **Yokota Masayuki.** Glucose sensor using a lead glass fiber polarization modulation device. *The 31st lightwave sensing technical study meeting LST 31-8,* August 2003, 51-56
- **Kajioka ; Oho.** Development of optical fiber gyro. *The third lightwave sensing technical study meeting, LST 3-9,* June 1989, 55-62